# EUROPEAN PATENT SPECIFICATION

(11) **EP 0 869 117 B1**
(45) Date of publication and mention of the grant of the patent: **30.08.2000**
(21) Application number: 98105495.0
(22) Date of filing: 01.04.1998
(51) Int. Cl.: C07C 311/08, C07C 215/08, A61K 31/18, A61K 9/08

(54) **Nimesulide choline salt, a process for the preparation thereof and pharmaceutical compositions containing it**
Cholin-Salz von Nimesulide, Verfahren zu seiner Herstellung und dieses enthaltende pharmazeutische Zusammensetzungen
Sel de choline de nimesulide, procédé pour sa préparation et compositions pharmaceutiques le contenant

(30) Priority: 01.04.1997 IT MI970751
(43) Date of publication of application: 07.10.1998
(73) Proprietor: Dompe' International S.A.M., 98000 Monaco (MC)
(72) Inventor: Di Schiena, Michele Giuseppe, 20080 Cisliano (MI) (IT); Di Schiena, Ivan Michele, 20080 Cisliano (MI) (IT)
(74) Representative: Minoja, Fabrizio, Dr.

(56) References cited:
- WO-A-95/34533
- US-A- 5 179 097

## Description

The invention relates to the nimesulide choline salt, i.e. (2-hydroxyethyl)trimethylammonium N-(4-nitro-2-phenoxyphenyl)methanesulfonamide, of formula (I) 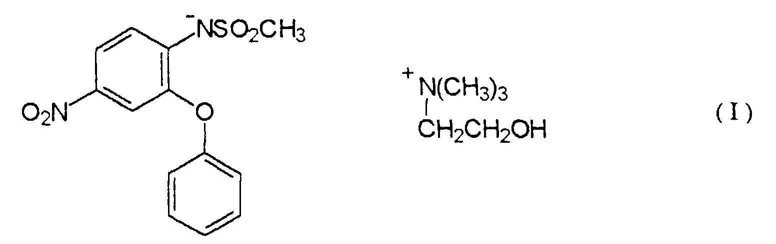 and to pharmaceutical compositions containing it having antiinflammatory, antiphlogistic, analgesic, antipyretic activities.

In particular, the invention relates to water-soluble pharmaceutical compositions and more particularly to water-soluble pharmaceutical compositions for the parenteral administration of nimesulide.

Nimesulide (The Merck Index 12^{th} edition) is a known antiinflammatory, antiphlogistic, analgesic and antipyretic drug, which differs from the most common non-steroidal antiinflammatory drugs (NSAD) in the sulfoanilide structure which gives it an acidic character.

Particularly remarkable is that nimesulide can be administered to patients with allergic hypersensitivity to aspirin or to other NSAD (Goodman & Gilman's, *THE PHARMACOLOGICAL BASIS OF THERAPEUTICS*, 9^{th} Ed.).

The drug is usually employed in solid pharmaceutical forms due to its remarkably low water solubility (about 0.01 mg/ml). This characteristic prevents the use of those water-soluble pharmaceutical forms in which a high concentration of nimesulide is required, such as injectable forms, drops, syrups, lotions and the like.

The known processes used in order to find a solution to the poor water-solubility of nimesulide generally involve the formation of salts or complexes; for example the sodium salt partially improves nimesulide water-solubility, which is anyway lower than 10 mg/ml; moreover, the sodium salt aqueous solutions are highly alkaline, and therefore scantily physiological.

Patents WO 91/17774 and 94/02177 disclose nimesulide complexes with cyclodextrins; said complexes, however, show only a poor increase in nimesulide water-solubility (about 0.05 mg/ml), involving no substantial advantages in the preparation of the above cited formulations, in which a high water-solubility of nimesulide is required.

Patent WO 95/34533 discloses nimesulide L-lysine salt, but also in this case water-solubility is not higher than 10 mg/ml; the addition of L-arginine in a remarkably high ratio, i.e. in a 1:1 ratio, being in fact necessary to obtain such a solubility value.

It has now been found, and this is the object of the present invention, that nimesulide choline salt overcomes the problems of the prior art described above. The compound of the invention, in fact, has surprising, peculiar chemical-physical and pharmacological properties, such as high water-solubility, up to 50% w/v, at room temperature and at a physiologically compatible pH, high stability of the aqueous solutions and high bioavailability; moreover choline, being a physiological compound, involves no problems of systemic or local toxicity; furthermore, the compound of the invention is present in the pharmaceutical compositions at a concentration much lower than the known posology.

The salt of the present invention can be administered in the form of conventional pharmaceutical formulations, particularly those requiring a high water-solubility of nimesulide, such as injectable forms, drops, syrups, granulates for extemporary reconstitution, effervescent forms, lotions, gels for the topical use, vaginal and urethral lavages, collutories, eye-drops, spray forms, controlled-release forms, transdermal delivery forms and systems, and the like.

The therapeutical fields which make use of the compound of the invention are, for example, internal medicine, traumatology, phlebology, odontostomatology, otorhinolaryngology, ophthalmology, urology, gynecology, proctology, dermatology.

Physiologically compatible compounds, capable of increasing the concentration of hydroxyl ions, can appropriately be used to increase the chemical stability in time of the aqueous solutions of the salt of the present invention.

A marked chemical stability in time of an aqueous solution of nimesulide choline salt at a concentration of 10 mg/ml up to 500 mg/ml, can be attained, for example, adding an alkaline carbonate, preferably sodium carbonate, in a concentration ranging from 2 mg/ml to 80 mg/ml; a preferred concentration of sodium carbonate ranging from 3 mg/ml to 20 mg/ml; a most preferred concentration of sodium carbonate being 10 mg/ml.

The posology of the compound of the present invention depends on the administration route, on the used pharmaceutical form, on the severity of the disease to treat and on the age of the patient. The parenteral dosage can range, for example, from 20 to 300 mg/day; the oral dosage can range from 10 to 400 mg/day; the rectal dosage can range from 50 to 500 mg/day; the dosage for the topical route depends on the type of formulation used, on the size and type of the area to treat and on the disease.

For example, in the case of a topical gel, dosage can be of 50 mg/administration; in the case of a collutory, it can be of 30 mg/administration.

The process for the preparation of the compound of the invention makes use of the known procedures for the preparation of salts.

For example, equimolecular ratios of nimesulide and choline hydroxide are reacted directly, preferably in water or in a suitable organic solvent, being it dry or variously hydrated, such as ethanol; the reaction temperature is not critical and it can be comprised in a wide range, a suitable temperature ranging from 15°C to 80°C.

The salt is recovered using known procedures, such as freeze-drying, crystallization, evaporation, precipitation with suitable solvents and the like.

In a particularly preferred embodiment of the invention, nimesulide is reacted with choline hydroxide in excess to the stoichiometric, using water as reaction solvent. In these conditions, nimesulide is salified completely. Water is evaporated off, then the residue is treated with 95% ethanol to crystallize the desired salt, which is highly pure, as the choline excess and the choline and nimesulide degradation products are completely removed.

The invention is further illustrated by the following examples.

### EXAMPLE 1

### Preparation of nimesulide choline salt

Nimesulide (61.6 g, 0.2 mols) is suspended in 250 ml of water and 35% w/w choline hydroxide in water (76.05 g, 0.22 mols) is added, stirring at 20°C, thereby obtaining an orange-red solution, which is stirred for 60 minutes, then filtered through paper. The clear solution is evaporated under reduced pressure in a rotary evaporator on a 60°C bath.

The solid residue is dissolved completely in 100 ml of 95% ethanol at 80°C and the resulting solution is left to crystallize, first at room temperature, then at 0°C for 10 hours.

The crystalline solid is filtered by suction and washed with 25 ml of ethanol for 4 times, then dried under vacuum at 60°C, to obtain 75.5 g (91.7% on theoretical) of nimesulide choline salt as an orange-yellow crystalline solid.
NMR: in agreement with the adscribed structure
m.p. = 133-135°C.

### EXAMPLE 2

### Parenteral preparation for extemporary reconstitution

Ampoule: nimesulide choline salt 66.7 mg (equivalent to 50 mg of nimesulide).

Solvent vial: distilled water for injections q.s. to 1 ml.

### EXAMPLE 3

### Parenteral preparation in vial

A vial contains: nimesulide choline salt 133.4 mg (equivalent to 100 mg of nimesulide), sodium carbonate 10 mg, distilled water for injections q.s. to 1 ml.

### EXAMPLE 4

### Oral drops

| **Composition** | **conc./100 ml** |
|---|---|
| Nimesulide | 4.00 g |
| Choline hydroxide | 1.57 g |
| Ethanol 95° | 35.50 g |
| Cellulose-polyethylene glycol ether | 0.50 g |
| Potassium acesulfame | 3.00 g |
| Flavour | 1.50 ml |
| Distilled water | 54.12 g. |

### EXAMPLE 5

### Collutory

| **Composition** | **conc./100 ml** |
|---|---|
| Nimesulide | 0.300 g |
| Choline hydroxide | 0.153 g |
| Ethanol 95° | 10.000 g |
| 70% Sorbitol | 50.000 g |
| Hydrogenated 40 (OE) castor oil | 1.500 g |
| Polyvinyl pyrrolidone K30 | 0.50 g |
| Cetyltrimetylammonium tosylate | 0.02 g |
| Potassium EDTA | 0.10 g |
| Potassium acesulfame | 0.50 g |
| Flavor | 0.25 ml |
| Depurated water | 48.49 g. |

### EXAMPLE 6

### Ear-drops

| **Composition** | **conc./100 ml** |
|---|---|
| Nimesulide choline salt | 3.00 g |
| Propylene glycol | 90.00 g |
| Distilled water | q.s. to 100.00 ml. |

### EXAMPLE 7

### Eye-drops

| **Composition** | **conc./100 ml** |
|---|---|
| Nimesulide choline salt | 1.000 g |
| Sodium chloride | 0.800 g |
| Polyvinyl alcohol | 1.400 g |
| Povidone | 0.500 g |
| Disodium EDTA | 0.013 g |
| Benzalkonium chloride | 0.004 g |
| Distilled water | q.s. to 100.00 ml. |

### EXAMPLE 8

### Nasal drops

| **Composition** | **conc./100 ml** |
|---|---|
| Nimesulide choline salt | 2.000 g |
| Timol | 0.010 g |
| Propylene glycol | 30.000 g |
| Polyethylene glycol stearate | 1.000 g |
| Povidone | 0.500 g |
| Depurated water | q.s. to 100.00 ml. |

## Claims

1. Compound of formula (I)

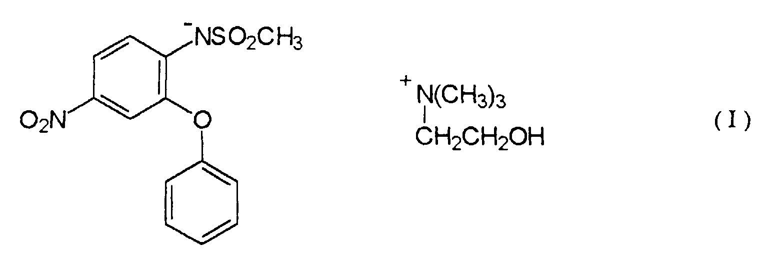

2. An aqueous solution containing the compound of formula (I).

3. A solution as claimed in claim 2, containing a physiologically compatible compound, capable of increasing the concentration of hydroxyl ions.

4. A solution as claimed in claim 3, wherein the physiologically compatible compound is sodium carbonate, potassium carbonate or lithium carbonate.

5. A solution as claimed in claim 4, wherein the concentration of alkaline carbonate ranges from 2 mg/ml to 80 mg/ml.

6. The compound as claimed in claim 1 as a therapeutical agent.

7. The use of the compound as claimed in claim 1, for the preparation of a medicament with antiinflammatory, antiphlogistic, analgesic, antipyretic activities.

8. Pharmaceutical compositions containing the compound as claimed in claim 1, in admixture with suitable excipients.

9. Pharmaceutical compositions as claimed in claim 8, in the form of aqueous solutions suitable for the oral, parenteral, rectal or topical administrations.

## Patentansprüche

1. Verbindung der Formel (I)

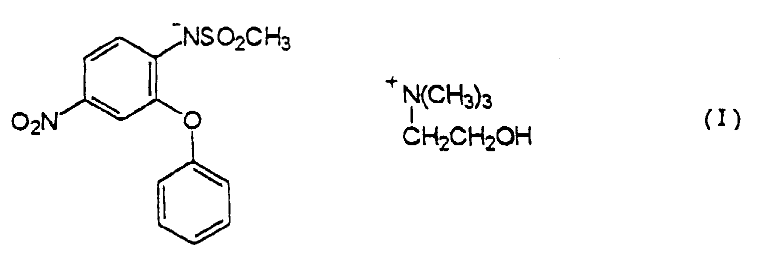

2. Wäßrige Lösung, enthaltend die Verbindung der Formel (I).

3. Lösung wie in Anspruch 2 beansprucht, enthaltend eine physiologisch verträgliche Verbindung, die zur Erhöhung der Konzentration von Hydroxylionen fähig ist.

4. Lösung wie in Anspruch 3 beansprucht, wobei die physiologisch verträgliche Verbindung Natriumcarbonat, Caliumcarbonat oder Lithiumcarbonat ist.

5. Lösung wie in Anspruch 4 beansprucht, wobei die Konzentration an Alkalicarbonat von 2 mg/ml bis 80 mg/ml reicht.

6. Verbindung wie in Anspruch 1 beansprucht als therapeutisches Mittel.

7. Verwendung der wie in Anspruch 1 beanspruchten Verbindung zur Herstellung eines Medikaments mit anti-entzündlichen, anti-phlogistischen, analgetischen, anti-pyretischen Aktivitäten.

8. Pharmazeutische Zusammensetzungen, enthaltend die Verbindung, wie in Anspruch 1 beansprucht, im Gemisch mit geeigneten Excipienten.

9. Pharmazeutische Zusammensetzungen wie in Anspruch 8 beansprucht, in Form von wäßrigen Lösungen, die geeignet sind für die orale, parenterale, rektale oder topische Gabe.

## Revendications

1. Composé de formule (I)

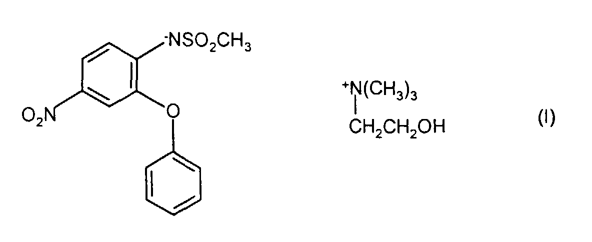

2. Solution aqueuse contenant le composé de formule (I).

3. Solution selon la revendication 2, contenant un composé physiologiquement compatible, capable d'accroître la concentration en ions hydroxyle.

4. Solution selon la revendication 3, dans laquelle le composé physiologiquement compatible est le carbonate de sodium, le carbonate de potassium ou le carbonate de lithium.

5. Solution selon la revendication 4, dans laquelle la concentration en carbonate alcalin est de 2 à 80 mg/ml.

6. Composé selon la revendication 1, en tant qu'agent thérapeutique.

7. Utilisation du composé selon la revendication 1 pour la préparation d'un médicament ayant des effets anti-inflammatoires, antiphlogistiques, analgésiques, antipyrétiques.

8. Compositions pharmaceutiques contenant le composé selon la revendication 1, en mélange avec des excipients appropriés.

9. Compositions pharmaceutiques selon la revendication 8, sous la forme de solutions aqueuses adaptées à des administrations par voie orale, parentérale, rectale ou topique.
